# EUROPÄISCHE PATENTSCHRIFT

(11) **EP 2 999 443 B1**
(45) Veröffentlichungstag und Bekanntmachung des Hinweises auf die Patenterteilung: **08.08.2018**
(21) Anmeldenummer: 14728440.0
(22) Anmeldetag: 16.05.2014
(51) Int. Cl.: A61F 13/02, A61F 13/00, A61K 9/70

(54) **PFLASTER MIT ABZIEHHILFE**
PLASTER COMPRISING PEELING AID
PATCH À PELAGE FACILITÉ

(30) Priorität: 23.05.2013 DE 102013008726
(43) Veröffentlichungstag der Anmeldung: 30.03.2016
(73) Patentinhaber: AMW GmbH, 83627 Warngau (DE)
(72) Erfinder: KAFFL, Hubert, 83627 Warngau (DE)
(74) Vertreter: Beckord & Niedlich Patentanwälte PartG mbB
(86) Internationale Anmeldenummer: PCT/EP2014/001332
(87) Internationale Veröffentlichungsnummer: WO 2014/187549

(56) Entgegenhaltungen:
- WO-A1-2012/041893
- WO-A2-2011/081810
- US-A- 6 149 614
- US-A1- 2006 151 347
- US-A1- 2012 006 710
- US-A1- 2012 283 614

## Beschreibung

Pflaster wie beispielsweise transdermale therapeutische Systeme weisen regelmäßig eine Folie auf, die die selbstklebende und gegebenenfalls wirkstoffhaltige Schicht schützt und vor der Applikation zu entfernen ist und als Abziehfolie bezeichnet wird. Es sind viele Vorschläge gemacht worden, das Abziehen der Folie zu erleichtern.

So ist die Abziehfolie gemäß EP 2 481 443 A1 ([0081] mit Figur 5A) mit einer Zeile von Einschnitten und gemäß Patentanmeldung JP HEI 8-112 305 (vgl. EP 1 552 821 B1 [0004]) oder EP 0 943 138 B1 ([0014]) mit einer Zeile von Perforationen versehen, so dass die Folie an der Zeile reißen und auseinander gezogen werden kann, um die selbstklebende Schicht freizugeben.

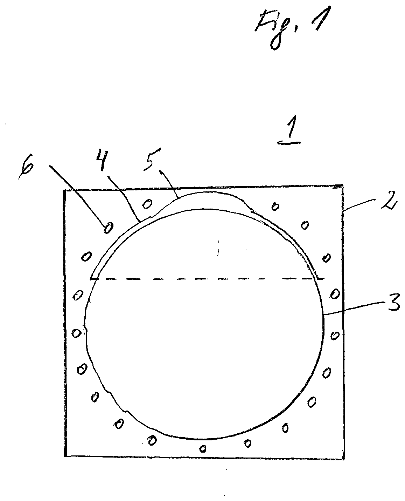

Auch hat man zwei Folien bzw. zwei Folienhälften vorgesehen, die gemeinsam die wirkstoffhaltige Schicht abdecken, jede Folie bzw. Folienhälfte für sich jedoch nur einen Teilbereich der selbstklebenden Schicht, beispielsweise etwa zur Hälfte, indem die abdeckende Folie mit einem halbierenden Schnitt ("half-cut") gehälftelt ist; vgl. EP 0 943 138 B1 ([0014]), EP 1 258 517 B1 ([0029] mit Figur 1), EP 1 411 906 B1 (Figur 2), EP 2 206 759 B1 (Figur 1), EP 2 481 443 A1 ([0082] mit Figur 5B). Sofern sich die beiden Folien überlappen, wird einem Austritt der Matrix der gegebenenfalls wirkstoffhaltigen Schicht, in der Regel eines selbsthaftenden Klebers, vorgebeugt; vgl. EP 1 180 023 B1 (Figur 1), EP 1 967 170 A1 (Figur 1.2), EP 2 340 815 B1 (Figur 4).

Immer wieder weist der Stand der Technik darauf hin, dass Matrix bzw. selbsthaftender Kleber durch die Abziehfolie austreten kann, sofern die Abziehfolie nicht geschlossen ist; vgl. EP 1 180 023 B1 (Spalte 2 Zeilen 46-50), EP 1 258 517 B1 ([0034], [0036], [0043]), EP 1 552 821 B1 ([0004], [0005], [0006]), EP 2 042 138 A1 ([0032]).

So hat man weiterhin vorgeschlagen, die Abziehfolie nicht mit einem Schnitt oder in Form von zwei Folienhälften vorzusehen, sondern die Abziehfolie nur mit einer Einkerbung zu versehen; vgl. EP 0 943 138 B1 ([0014]), EP 1 258 517 B1 ([0036] mit Figur 2 und [0042] mit Figur 4), EP 1 552 821 B1 ([0015] mit Figuren 1-2), EP 1 915 976 A1 ([0010]), EP 2 042 138 A1 ([0031] mit Figur 1), EP 2 078 517 A2 ([0016] mit Figur 2) und EP 2 489 340 A1 ([0067] mit Figur 5).

EP 0 943 138 B1 beschreibt ein Pflaster mit Abdeckfolie, Klebstoffschicht und Abziehfolie, wobei die Abziehfolie Einkerbungen als Sollbruchlinien aufweisen kann ([0014]), wobei die Sollbruchlinien von unten bzw. von der Seite der Abziehfolie her eingestanzt werden, die der Klebstoffschicht abgewandt ist (Seite 2 links Zeilen 31-34).

Nach EP 1 258 517 B1 wird ein Klebelement aus einem Träger bzw. einer Deckfolie (1), Klebschicht (2) und Abziehfolie (3) mit einem Einschnitt ("half-cut") vorgesehen, der als hälftelnder Schnitt oder Einkerbung ausgebildet sein kann (Ansprüche 9, 11 und 13). Der hälftelnde Schnitt bzw. die Einkerbung kann grundsätzlich bis zu einer Tiefe vorgesehen werden, die mehr als 14/15 der Dicke der Abziehfolie beträgt, jedoch weniger als deren Dicke plus der Dicke der Klebschicht (Anspruch 2). Die Einkerbung wird also von der der Klebeschicht abgewandten Seite der Abziehfolie her vorgenommen, so dass die Einkerbung von da her bis in die Klebeschicht eindringen kann. Die Einkerbung muss aber nicht bis in die Klebeschicht geführt werden, sie kann auch mit einer Tiefe von weniger als der Dicke der Abziehfolie, jedoch mehr als 14/15 ihrer Dicke vorgesehen werden.

EP 1 552 821 B1 ([0015] und [0017] mit Figuren 1-2) beschreibt eine Abziehfolie für ein Pflaster aus dieser Folie, einer Grundmittelschicht und einem Träger. Dabei ist die Abziehfolie mit einem Kantenabschnitt eines Presselements in einem Abschnitt, dem sogenannten Abreißlinien-Abschnitt, derart verpresst, dass die Stärke des verpressten Abschnitts geringer ist als die Stärke der Abziehfolie (Ansprüche 1 und 5). So kann eine PE-Abziehfolie eines Pflasters aus der Folie, einem Salbenauftrag und einem abdeckenden Gewebe mit einem heißen Heißsiegel-Draht in Berührung gebracht und der sogenante Abreißlinien-Abschnitt vorgesehen werden (Beispiel 5).

EP 1 915 976 A1 ([0010]) beschreibt ein Pflaster mit einer Abziehfolie, die eine Trennzone aufweist, die einen Bereich mit einem dünnen Schnitt bzw. dünner Schnitte ("thin cut part") aufweisen kann, die nicht durch die Abziehfolie hindurch geführt sind, bei denen es sich um eine Einkerbung ("half-cut") handeln kann ([0010]), wobei benachbart zur Trennzone zusätzlich ein oder mehrere vorgeschnittene Bereiche ("precut part") vorgesehen sein können, bei denen es sich gleichfalls um Einkerbungen handeln kann [0013]. Stand der Technik, nach dem die Abziehfolie eines Pflasters mit Einkerbungen versehen werden kann, wird vorausgesetzt.

EP 2 042 138 A1 beansprucht ein Pflaster mit Deckfolie, Klebschicht und Abziehfolie, bei dem die Abziehfolie mit einer der Klebschicht abgewandten Einkerbung einer Tiefe versehen ist, die mehr als die halbe Dicke, aber weniger als die Dicke der Folie misst (Anspruch 1).

EP 2 078 517 A2 beansprucht ein Verfahren zur Verwendung eines derartigen Pflasters (Ansprüche 1 bis 5) sowie ein in dem Verfahren zu verwendendes Pflaster (Anspruch 6).

EP 2 489 340 A1 beansprucht ein Klebepflaster mit einer Trägerfolie, einer Klebemittelschicht und einer Abziehfolie (16 in Figur 5), wobei die Abziehfolie eine Schwachzone (20 in Figur 5) in Form einer Kerbe ("half-cut" gemäß [0067]) aufweist, und ferner eine Folie (18 in Figur 5) mit einer Schwachzone (gleichfalls 20 in Figur 5) trägt, die entsprechend der Schwachzone der Abziehfolie positioniert ist und mit einem Teilbereich auf der Abziehfolie fixiert ist und mit einem anderen Teilbereich als Griff (bzw. pinching piece) dient (18a, 18b in Figur 1). Indem die Folie hautseitig bzw. applikationsseitig auf der Abziehfolie vorgesehen ist, wird sie zusammen mit der Abziehfolie entfernt. Die Schwachzonen (20 in Figur 5) können dadurch vorgesehen werden, dass die Abziehfolie mit der von ihr getragenen weiteren Folie einem Laser ausgesetzt wird ([0067]).

EP 1 231 906 B1 beschreibt ein Pflaster mit einem Reservoir (2), das einen leichtflüchtigen Wirkstoff enthält, und das mit einem Verbund aus einer wirkstoffdurchlässigen Polymermembran (5) und einer wirkstoffundurchlässigen Polyesterfolie (9) abgedeckt ist. Hautseitig ist das Reservoir (2) durch eine Dichtfolie (6) verschlossen, die eine Haftklebeschicht (7) trägt, die vor der Applikation durch eine abziehbare Schutzschicht (1) geschützt ist. Die Polyesterfolie (9) kann eine Abziehhilfe bzw. ein Klebeetikett (4) tragen, mit denen nach Applikation des Pflasters die Polyesterfolie (9) von der Polymermembran (5) abgezogen werden kann, um den leichtflüchtigen Wirkstoff für eine inhalative Aufnahme freizusetzen. Eine Abziehhilfe zum Freilegen der Haftklebeschicht ist auch diesem Stand der Technik fremd.

Soll ein Pflaster eingesetzt werden, von dessen selbstklebender Schicht die Abziehfolie in Form von zwei Teilen abzuziehen ist, so wird das Pflaster längs der Stanzung (12), die die Folie durchzieht, derart gebogen oder gefaltet, dass die Stanzung (12) im Bug der Aufbiegung oder Auffaltung zu liegen kommt und die selbstklebende Schicht in der schnittartigen Stanzung oder nach dem Bruch der Stanzung spaltförmig freigelegt wird. Danach kann einer der am Bug aufstehenden Teile der Abziehfolie ergriffen und abgezogen werde, wonach der freigelegte Bereich der selbstklebenden Schicht haftend auf die Haut geklebt werden kann. Indem der freie, noch nicht verklebte Flügel des Pflasters weiter gebogen wird, löst sich auch von diesem Flügel der noch verbliebene Teil der Abziehfolie so weit ab, dass auch er ergriffen und abgezogen werden kann. Nun läßt sich der jetzt freigelegte restliche Bereich der selbstklebenden Schicht haftend auf die Haut aufkleben. Für alle diese Schritte ist eine nicht unbeträchtliche manuelle Geschicklichkeit erforderlich, die vor allem von Kindern und älteren Menschen schlecht erbracht werden kann.

Der Erfindung liegt nun die Aufgabe zugrunde, die Entfernbarkeit der Abziehfolie bei der Applikation eines transdermalen Systems zu verbessern und möglichst auch die Gefahr zu minimieren, dass Klebemittel bzw. Matrix und gegebenenfalls Wirkstoff bei der Lagerung eines transdermalen Pflasters austreten.

Die der Erfindung zugrunde liegende Aufgabe wird nun durch ein Pflaster gelöst, insbesondere ein transdermales vorzugsweise therapeutisches System, mit
- einer Deckfolie,
- einer Schicht (Matrix-Schicht), bestehend aus einer oder mit einer Matrix, die wirkstoffhaltig sein kann, und
- einer einteiligen Abziehfolie,
   (i) wobei die Matrix der Matrix-Schicht selbstklebend ist und mit einer selbstklebenden Klebemittel-Schicht versehen sein kann, die zwischen der Matrix-Schicht und der Abziehfolie vorgesehen ist, wobei ferner zwischen der Matrix-Schicht, sofern eine Klebemittel-Schicht fehlt, oder zwischen der Klebemittel-Schicht und der Abziehfolie eine Abziehhilfe vorgesehen ist,
      oder
   (ii) wobei die Matrix der Matrix-Schicht nicht-selbstklebend ist und mit einer selbstklebenden Klebemittel-Schicht versehen ist, die zwischen der Matrix-Schicht und der Abziehfolie vorgesehen ist, wobei ferner zwischen der Klebemittel-Schicht und der Abziehfolie eine Abziehhilfe vorgesehen ist,
   wobei die Abdeckfolie, Matrix-Schicht und fakultative Klebemittel-Schicht als Bauelemente des Pflasters die Form von deckungsgleichen Scheiben aufweisen und ferner
- die Abziehhilfe als flaches weiteres Bauelement ausgebildet sein,
- die Abziehhilfe weniger als die halbe Fläche der Matrix-Schicht oder der Klebemittel-Schicht abdeckt, und
- die Abziehhilfe mit einem Griff, einer Ausbuchtung oder einer Nase über die Peripherie der Matrix-Schicht oder die Klebemittel-Schicht vorragt.

Erfindungsgemäß ist also die Abziehfolie einteilig. Sie ist also weder mit Perforationen oder Einschnitten, also auch nicht mit sogenannten "half-cuts" oder "precut parts" versehen. Dadurch wird verhindert, dass Matrix durch die Abziehfolie austritt, was insbesondere während der Lagerung von Pflastern wie z.B. transdermalen Systemen unerwünscht ist.

Erfindungsgemäß kann die Abziehhilfe nicht nur dazu dienen, das Abziehen der Abziehfolie zu erleichtern, sie kann auch bei der Applikation des von der Abziehfolie befreiten Pflasters als Applikations- und Spendehilfe dienlich sein.

Die Scheiben können beispielsweise kreisförmig oder elliptisch sein.

Ferner können bei dem erfindungsgemäßen Pflaster, insbesondere transdermalen vorzugsweise therapeutischen System Abdeckfolie, Matrix-Schicht und fakultative Klebemittel-Schicht als Bauelemente des Pflasters die Form von Kreisscheiben aufweisen und ferner
- die Abziehhilfe in Form eines Segments der Kreisscheiben vorgesehen sein oder
- die Abziehhilfe in Form eines Vektors der Kreisscheiben vorgesehen sein oder
- die Abziehhilfe als Streifen ausgebildet sein, wobei
die an der Peripherie der Matrix-Schicht oder der Klebemittel-Schicht gelegene gekrümmte Peripherie des Segments oder des Vektors oder Schmalseite des Streifens mit der Peripherie der Kreisscheiben fluchtet.

Ferner kann bei dem erfindungsgemäßen Pflaster, insbesondere transdermalen vorzugsweise therapeutischen System der Griff ein Fingergriff sein, vorzugsweise in Form einer Ausbuchtung oder Nase.

Ferner kann erfindungsgemäß ein Pflaster, insbesondere transdermales vorzugsweise therapeutisches System vorgesehen werden,
- bei dem die Abziehhilfe in Form eines Segments der Kreisscheiben vorgesehen ist und kleiner als ein Halbkreissegment ist oder
- bei dem die Abziehhilfe in Form eines Vektors der Kreisscheiben vorgesehen ist und kleiner als ein Halbkreisvektor ist oder
- bei dem die Abziehhilfe als Streifen ausgebildet ist, der kürzer als der Radius der Kreisscheiben ist, und
wobei die an der Peripherie der Matrix-Schicht oder der Klebemittel-Schicht gelegene gekrümmte Peripherie des Segments oder des Vektors oder die Schmalseite des Streifens einen Griff, eine Ausbuchtung oder eine Nase aufweist, die die Peripherie überragen.

Bei einem Streifen kann es sich um eine Abziehhilfe handeln, bei der der Streifen etwa parallele, insbesondere gerade seitliche Begrenzungen und eine Länge aufweist, die kürzer ist als der Radius der Kreisscheiben der Abdeckfolie, Matrix-Schicht und fakultativen Klebemittel-Schicht, wobei der Streifen mit einem Griff oder einer Nase die Peripherie der Matrix-Schicht oder die Klebemittel-Schicht überragen kann.

Ferner kann bei dem erfindungsgemäßen Pflaster, insbesondere transdermalen vorzugsweise therapeutischen System die Abziehhilfe auf der der Abziehfolie abgewandten Seite mit einem Trennmittel versehen sein, vorzugsweise silikonisiert oder metallbedampft sein. Auf der der Abziehfolie zugewandten Seite kann die Abziehfolie mit einem Klebemittel versehen sein.

Ferner kann bei dem erfindungsgemäßen Pflaster, insbesondere transdermalen vorzugsweise therapeutischen System die Abziehhilfe aus demselben Material bestehen wie die Abziehfolie oder die Deckfolie. Für geeignete Materialien kann auf den eingangs angeführten Stand der Technik verwiesen werden. Ein Beispiel für ein geeignetes Material ist Polyethylenterephthalat.

Ferner kann bei dem erfindungsgemäßen Pflaster, insbesondere transdermalen vorzugsweise therapeutischen System die Abziehfolie auf der hautabgewandten Seite mit einem Trennmittel versehen sein, vorzugsweise silikonisiert oder metallbedampft sein. Sie kann über ihre gesamte Fläche dieselbe Stärke aufweisen.

Ferner kann bei dem erfindungsgemäßen Pflaster, insbesondere transdermalen vorzugsweise therapeutischen System die Abziehfolie die Deckfolie mit Matrix-Schicht und fakultativer oder obligatorischer Klebemittel-Schicht allseitig überragen.

Ein erfindungsgemäßes Pflaster kann für schützende, kosmetische oder therapeutische Zwecke vorgesehen werden. Es kann also einen oder mehrere Wirkstoffe enthalten.

Ein erfindungsgemäßes Pflaster, insbesondere transdermales vorzugsweise therapeutisches System kann dadurch hergestellt werden, dass auf einem Band, aus dem die Abziehfolien der herzustellenden transdermalen Systeme separiert werden, in Abständen zuerst Abziehhilfen und danach auf jede einzelne Abziehhilfe ein Verbund aus Deckfolie mit Matrix-Schicht und fakultativer Klebemittel-Schicht abgelegt werden, wonach aus dem Band für jeden Verbund mit seiner Abziehhilfe einzelne Abziehfolien bzw. Pflaster separiert werden.

**Figur 1** zeigt in Draufsicht auf ein erfindungsgemäßes Pflaster 1 mit einer quadratischen Abziehfolie 2, auf die mittig eine kreisförmige Deckfolie 3 aufgebracht ist, die vollflächig mit einem selbstklebenden und auf der Abziehfolie 2 klebenden Kleber beschichtet ist. Darunter kann eine selbstklebende oder nicht-selbstklebende Matrix-Schicht mit einer fakultativen selbstklebenden Klebemittel-Schicht zu verstehen sein. Zwischen der selbstklebenden Kleber-Schicht und der Abziehfolie 2 ist eine Abziehhilfe 4 vorgesehen, die die Form eines Segments der kreisförmigen Deckfolie 3 aufweist und deren gekrümmte Peripherie sich nahezu mit der Peripherie der Deckfolie 3 deckt. Die Abziehhilfe 4 ist mit einem Griff in Form einer konvexen Ausbuchtung 5 versehen, die über die Peripherie der Deckfolie 3 vorragt. Allgemein läßt sich sagen, dass der Griff die Abziehfolie nicht überragen sollte. Ebenso wie er (wie dargestellt) mittig auf eine Längsseite der Abziehfolie 2 weist, kann er auch auf eine Ecke der Abziehfolie 2 weisen, wodurch er noch weiter über die Peripherie der Deckfolie 3 vorragen kann.
Ein erfindungsgemäßes Pflaster kann allgemein auch mit Noppen 6 versehen sein, die die Deckfolie 3 mit Matrix-Schicht und fakultativer Klebemittel-Schicht im Abstand umziehen können und die Deckfolie 3 überragen, um eine eventuelle Verpackung (nicht dargestellt) insbesondere von der Peripherie der Deckfolie 3 mit Matrix-Schicht und fakultativer Klebemittel-Schicht bzw. von Matrix oder Klebemittel abzuhalten, die aus dem Ringspalt zwischen Deckfolie 3 und Abziehfolie 4 bei längerer Lagerung ausgetreten sein könnten.
Bei Applikation eines erfindungsgemäßen Pflasters läßt sich die Abziehhilfe 4 leicht am Griff bzw. der Ausbuchtung 5 mit zwei Fingern fassen, anheben und zusammen mit der Deckfolie 3 mit Matrix-Schicht und fakultativer Klebemittel-Schicht vollständig von der mit einem Trennmittel versehenen Abziehfolie 2 abheben. Danach kann der Verbund aus Abziehhilfe 4, Deckfolie 3, Matrix-Schicht und fakultativer Klebemittel-Schicht mit Hilfe der Abziehhilfe als Applikations- bzw. Spendehilfe auf die Haut in dem Bereich der Matrix-Schicht und der fakultativen Klebemittel-Schicht aufgeklebt werden, der nicht durch die Abziehhilfe abgedeckt ist. Schließlich kann die gegebenenfalls mit einem Trennmittel versehene Abziehhilfe derart aufgebogen werden, dass sie sich an der dem Griff bzw. der Ausbuchtung 5 gegenüberliegenden Sehne von der Matrix-Schicht und der fakultativen Klebemittel-Schicht bis an deren Peripherie löst und sich nun leicht abheben läßt, während sich das Pflaster jetzt vollflächig auf der Haut verkleben läßt.

### Beispiel 1

Auf einem einseitig silikonisierten Endlosband aus Polyethylenterephthalat einer Dicke von 75 Mikrometer und einer Breite von 50 mm wurden in Abständen von 50 mm Abziehhilfen abgelegt. Diese Abziehhilfen hatten die Form eines Segments eines Kreises, dessen Durchmesser 39 mm betrug. Die Sehne jeder Abziehhilfe war 37 mm lang. Auf der der Sehne gegenüberliegenden Seite jeder Abziehhilfe war als Fingergriff eine konvexe Ausbuchtung vorgesehen, die mittig mit ihrem Scheitel um 2 mm über die teilkreisförmige Peripherie jedes Segmentes bzw. jeder Abziehhilfe vorragte. Diese Ausbuchtungen der abgelegten Abziehhilfen lagen jeweils in der Flucht des Endlosbandes, und zwar jeweils in dessen Förderrichtung.

Zusätzlich wurden Deckfolien in Form von Kreisscheiben aus Polyethylenterephthalat einer Dicke von 75 Mikrometer und eines Durchmessers von 39 mm vorgesehen, die flächendeckend mit einem Haftkleber (z.B. DuroTak 6911A) einer Schichtdicke von 40 g (Trockengewicht)/m² beschichtet waren.

Auf jeder Abziehhilfe wurde eine beschichtete Deckfolie derart abgelegt, dass sich die Peripherie der beschichteten Deckfolie und die teilkreisförmige Peripherie der Abziehhilfe deckten, wobei die Abziehhilfe mit ihrer Ausbuchtung über die Peripherie der Deckfolie vorragte.

Danach wurden aus dem Endlosband Quadrate einer Kantenlänge von 47 mm derart gestanzt, dass jedes Quadrat als Abziehfolie eine beschichtete Deckfolie mit Abziehhilfe trug.

### Beispiel 2

Für die Herstellung eines erfindungsgemäßen Pflasters wurden ausgestanzte Folienstücke eines Prozessliners herangezogen, der als Prozessfolie bzw. Hilfs-Abziehfolie bei der Herstellung eines wirkstoffhaltigen Laminats diente. Die ausgestanzten Folienstücke wurden jeweils zusammen mit einer wirkstoffhaltigen Klebematrix und einer Deckfolie auf die finale Abziehfolie (Release Liner) aufgespendet bzw. aufkaschiert, wonach zur Herstellung der Pflaster formgebend gestanzt wurde.

## Patentansprüche

1. Pflaster (1) mit
- einer Deckfolie (3),
- einer Schicht (Matrix-Schicht), bestehend aus einer oder mit einer Matrix, die wirkstoffhaltig sein kann, und
- einer einteiligen Abziehfolie (2),
(i) wobei die Matrix der Matrix-Schicht selbstklebend ist und mit einer selbstklebenden Klebemittel-Schicht versehen sein kann, die zwischen der Matrix-Schicht und der Abziehfolie (2) vorgesehen ist, wobei ferner zwischen der Matrix-Schicht, sofern eine Klebemittel-Schicht fehlt, oder zwischen der Klebemittel-Schicht und der Abziehfolie (2) eine Abziehhilfe (4) vorgesehen ist,
oder
(ii) wobei die Matrix der Matrix-Schicht nicht-selbstklebend ist und mit einer selbstklebenden Klebemittel-Schicht versehen ist, die zwischen der Matrix-Schicht und der Abziehfolie (2) vorgesehen ist, wobei ferner zwischen der Klebemittel-Schicht und der Abziehfolie (2) eine Abziehhilfe (4) vorgesehen ist,
wobei die Abdeckfolie (3), Matrix-Schicht und fakultative Klebemittel-Schicht als Bauelemente des Systems die Form von deckungsgleichen Scheiben aufweisen und ferner
- die Abziehhilfe (4) als flaches weiteres Bauelement ausgebildet ist,
- die Abziehhilfe (4) weniger als die halbe Fläche der Matrix-Schicht oder der Klebemittel-Schicht abdeckt, und
die Abziehhilfe (4) mit einem Griff, einer Ausbuchtung oder einer Nase (5) über die Peripherie der Matrix-Schicht oder die Klebemittel-Schicht vorragt.

2. Pflaster (1) nach Anspruch 1, bei dem Abdeckfolie (3), Matrix-Schicht und fakultative Klebemittel-Schicht als Bauelemente des Systems die Form von Kreisscheiben aufweisen und ferner
- die Abziehhilfe (4) in Form eines Segments der Kreisscheiben vorgesehen ist oder
- die Abziehhilfe (4) in Form eines Vektors der Kreisscheiben vorgesehen ist oder
- die Abziehhilfe (4) als Streifen ausgebildet ist, wobei
die an der Peripherie der Matrix-Schicht oder der Klebemittel-Schicht gelegene gekrümmte Peripherie des Segments oder des Vektors oder Schmalseite des Streifens mit der Peripherie der Kreisscheiben fluchtet.

3. Pflaster (1) nach Anspruch 1 oder 2, bei dem der Griff ein Fingergriff in Form einer Ausbuchtung oder Nase (5) ist.

4. Pflaster (1) nach einem der Ansprüche 1, 2 oder 3,
- bei dem die Abziehhilfe (4) in Form eines Segments der Kreisscheiben vorgesehen ist und kleiner als ein Halbkreissegment ist oder
- bei dem die Abziehhilfe (4) in Form eines Vektors der Kreisscheiben vorgesehen ist und kleiner als ein Halbkreisvektor ist oder
- bei dem die Abziehhilfe (4) als Streifen ausgebildet ist, der kürzer als der Radius der Kreisscheiben ist, und
wobei die an der Peripherie der Matrix-Schicht oder die Klebemittel-Schicht gelegene gekrümmte Peripherie des Segments oder des Vektors oder Schmalseite des Streifens einen Griff oder eine Nase (5) aufweist, die die Peripherie überragen.

5. Pflaster (1) nach einem der Ansprüche 1, 2, 3 oder 4, bei dem die Abziehhilfe (4) auf der der Abziehfolie (2) abgewandten Seite mit einem Trennmittel versehen ist.

6. Pflaster (1) nach einem der Ansprüche 1, 2, 3, 4 oder 5, bei dem die Abziehhilfe (4) aus demselben Material besteht wie die Abziehfolie (2) oder die Deckfolie (3).

7. Pflaster (1) nach einem der Ansprüche 1, 2, 3, 4, 5 oder 6, bei dem die Abziehfolie (2) über ihre gesamte Fläche dieselbe Stärke aufweist.

8. Pflaster (1) nach mindestens einem der vorhergehenden Ansprüche, bei dem die Abziehfolie (2) die Deckfolie (3) mit Matrix-Schicht und fakultativer oder obligatorischer Klebemittel-Schicht allseitig überragt.

## Claims

1. A plaster (1) having
- a cover film (3),
- a layer (matrix layer) consisting of or having a matrix which can contain active agents, and
- a single-part release liner (2),
(i) wherein the matrix of the matrix layer is self-adhesive and can be provided with a self-adhesive, adhesive agent layer which is provided between the matrix layer and the release liner (2), wherein a pull-off aid (4) is further provided between the matrix layer, if an adhesive agent layer is omitted, or between the adhesive agent layer and the release liner (2),
or
(ii) wherein the matrix of the matrix layer is not self-adhesive and is provided with a self-adhesive, adhesive agent layer which is provided between the matrix layer and the release liner (2), wherein a pull-off aid (4) is also provided between the adhesive agent layer and the release liner (2),
wherein the top film (3), matrix layer and optional adhesive agent layer as structural elements of the system are in the form of congruent discs and also
- the pull-off aid (4) is in the form of a flat further structural element,
- the pull-off aid (4) covers less than half the area of the matrix layer or of the adhesive agent layer, and
the pull-off aid (4) protrudes with a grip, a bulge or a nib (5) beyond the periphery of the matrix layer or the adhesive agent layer.

2. Plaster (1) according to Claim 1, wherein the top film (3), matrix layer and optional adhesive agent layer as structural elements of the system are in the form of circular discs and also
- the pull-off aid (4) is provided in the form of a segment of the circular discs, or
- the pull-off aid (4) is provided in the form of a vector of the circular discs, or
- the pull-off aid (4) is in the form of a strip, wherein
the curved periphery of the segment or of the vector or short side of the strip at the periphery of the matrix layer or of the adhesive agent layer alignes with the periphery of the circular discs.

3. Plaster (1) according to Claim 1 or 2, wherein the grip is a finger grip in the form of a bulge or nib (5).

4. Plaster (1) according to any one of Claims 1, 2 or 3,
- wherein the pull-off aid (4) is provided in the form of a segment of the circular discs and is smaller than a semi-circular segment, or
- wherein the pull-off aid (4) is provided in the form of a vector of the circular discs and is smaller than a semi-circular vector, or
- wherein the pull-off aid (4) is in the form of a strip which is shorter than the radius of the circular discs, and
wherein the curved periphery of the segment or of the vector or short side of the strip at the periphery of the matrix layer or the adhesive agent layer has a grip or a nib (5) which protrudes beyond the periphery.

5. Plaster (1) according to any one of Claims 1, 2, 3 or 4, wherein the pull-off aid (4) is provided with a release agent on the side facing away from the release liner (2).

6. Plaster (1) according to any one of Claims 1, 2, 3, 4 or 5, wherein the pull-off aid (4) consists of the same material as the release liner (2) or the cover film (3).

7. Plaster (1) according to any one of Claims 1, 2, 3, 4, 5 or 6, wherein the release liner (2) has the same strength over its entire area.

8. Plaster (1) according to at least one of the preceding claims, wherein the release liner (2) protrudes beyond the cover film (3) with the matrix layer and the optional or obligatory adhesive agent layer, on all sides.

## Revendications

1. Sparadrap (1) comprenant
- un film de couverture (3),
- une couche (couche matricielle), constituée d'une matrice ou comprenant une matrice, qui peut contenir des principes actifs et
- un film détachable (2) en un élément,
(i) la matrice de la couche matricielle étant autocollante et pouvant être munie d'une couche d'agent adhésif autocollant qui est prévue entre la couche matricielle et le film détachable (2), alors que par ailleurs, entre la couche matricielle, si une couche d'agent adhésif fait défaut, ou entre la couche d'agent adhésif et le film détachable (2), il est prévu une aide au retrait (4),
ou
(ii) la matrice de la couche matricielle n'étant pas autocollante et étant munie d'une couche d'agent adhésif autocollant, qui est prévue entre la couche matricielle et le film détachable (2) alors que par ailleurs, entre la couche d'agent adhésif et le film détachable (2), il est prévu une aide au retrait (4),
en tant qu'éléments constitutifs du système, le film de couverture (3), la couche matricielle et la couche d'agent adhésif facultative présentant la forme de disques coïncidentes et par ailleurs,
- l'aide au retrait (4) étant conçue sous la forme d'un élément constitutif supplémentaire plat,
- l'aide au retrait (4) recouvrant moins de la demi-surface de la couche matricielle ou de la couche d'agent adhésif et
- l'aide au retrait (4) saillant par une poignée, un creux ou un tenon (5) par-dessus la périphérie de la couche matricielle ou de la couche d'agent adhésif.

2. Sparadrap (1) selon la revendication 1, sur lequel en tant qu'éléments constitutifs du système, le film de couverture (3), la couche matricielle et la couche d'agent adhésif facultative présentent la forme de disques circulaires et par ailleurs,
- l'aide au retrait (4) est prévue sous la forme d'un segment des disques circulaires ou
- l'aide au retrait (4) est prévue sous la forme d'un vecteur des disques circulaires ou
- l'aide au retrait (4) est conçue en tant que ruban,
la périphérie curviligne du segment ou du vecteur ou le côté étroit du ruban situé(e) sur la périphérie de la couche matricielle ou de la couche d'agent adhésif étant alignée sur la périphérie des disques circulaires.

3. Sparadrap (1) selon la revendication 1 ou 2, sur lequel la poignée est un onglet sous la forme d'un creux ou d'un tenon (5).

4. Sparadrap (1) selon l'une quelconque des revendications 1, 2 ou 3,
- sur lequel l'aide au retrait (4) est prévue sous la forme d'un segment des disques circulaires et est plus petite qu'un segment semi-circulaire ou
- sur lequel l'aide au retrait (4) est prévue sous la forme d'un vecteur des disques circulaires et est plus petite qu'un vecteur semi-circulaire ou
- sur lequel l'aide au retrait (4) est conçue en tant que ruban, qui est plus court que le rayon des disques circulaires et
la périphérie curviligne du segment ou du vecteur ou le côté étroit du ruban situé(e) sur la périphérie de la couche matricielle ou de la couche d'agent adhésif comportant une poignée ou un tenon (5) qui saillissent par-dessus la périphérie.

5. Sparadrap (1) selon l'une quelconque des revendications 1, 2, 3 ou 4, sur lequel sur le côté opposé au film détachable (2), l'aide au retrait (4) est munie d'un moyen de sectionnement.

6. Sparadrap (1) selon l'une quelconque des revendications 1, 2, 3, 4 ou 5, sur lequel l'aide au retrait (4) est composée de la même matière que le film détachable (2) ou le film de couverture (3).

7. Sparadrap (1) selon l'une quelconque des revendications 1, 2, 3, 4, 5 ou 6, sur lequel le film détachable (2) présente la même épaisseur sur toute sa surface.

8. Sparadrap (1) selon au moins l'une quelconque des revendications précédentes, sur lequel le film détachable (2) saillit de toutes parts par-dessus le film de couverture (3) avec la couche matricielle et la couche d'agent adhésif obligatoire ou facultative.
